# EUROPEAN PATENT APPLICATION

(11) **EP 4 310 717 A1**
(43) Date of publication of application: **24.01.2024**
(21) Application number: 22770965.6
(22) Date of filing: 14.02.2022
(51) Int. Cl.: G06F 30/27, G16Z 99/00

(54) **RECOMMENDATION DATA GENERATION DEVICE, CONTROL METHOD, AND NON-TRANSITORY COMPUTER-READABLE MEDIUM**

(30) Priority: 18.03.2021 JP 2021044485
(71) Applicant: NEC Corporation, Tokyo 108-8001 (JP); Tohoku University, Sendai-shi, Miyagi 980-8577 (JP)
(72) Inventor: KUWAMORI, Naoki, Tokyo 108-8001 (JP); MUSA, Akihiro, Tokyo 108-8001 (JP); KAZAMA, Yuka, Tokyo 108-8001 (JP); TAKIGAWA, Yohei, Tokyo 108-8001 (JP); SATOU, Yoshihiko, Tokyo 136-8627 (JP); KOBAYASHI, Hiroaki, Sendai-shi, Miyagi 980-8577 (JP); KIKUGAWA, Gota, Sendai-shi, Miyagi 980-8577 (JP); OKABE, Tomonaga, Sendai-shi, Miyagi 980-8577 (JP); KOMATSU, Kazuhiko, Sendai-shi, Miyagi 980-8577 (JP)
(74) Representative: Betten & Resch
(86) International application number: PCT/JP2022/005623
(87) International publication number: WO 2022/196206

(57) **Abstract**

A recommendation data generation apparatus (2000) acquires material specification information (10) representing a material specification of a material (60) and physical property information (20) representing a physical property of a product (70). The recommendation data generation apparatus (2000) generates a self-organizing map (30) by using the physical property information (20). Each node on the self-organizing map (30) is assigned a position in a map space and a physical property vector representing a physical property. The recommendation data generation apparatus (2000) assigns each node a specification vector representing the material specification by using the material specification information (10). The recommendation data generation apparatus (2000) acquires target information (80) representing a desired physical property, and detects a target node to which a physical property vector matching that physical property is assigned. The recommendation data generation apparatus (2000) generates, by using the specification vector assigned to the target node, recommendation data (90) representing the material specification with which a product (70) having a desired physical property can be generated.

## Description

### Technical Field

The present disclosure relates to a technology for providing information related to product development.

### Background Art

In product development, there are cases to conduct a search for a material with which a product having desired properties can be generated. Therefore, systems for helping a user or the like search for such a material have been developed. For example, Patent Literature 1 discloses a system for helping a use or the like design a tire by using a self-organization map.

### Citation List

### Patent Literature

Patent Literature 1: Japanese Unexamined Patent Application Publication No. 2016-148988

### Summary of Invention

### Technical Problem

In Patent Literature 1, the self-organizing map is used to determine which one of a plurality of design variables of the tire is/are important factor. Therefore, it is not assumed that the self-organizing map is used for any purpose other than the above-described purpose. The present disclosure has been made in view of the above-described problem, and an objective thereof is to provide a new technique for providing information useful for product development.

### Solution to Problem

A recommendation data generation apparatus according to the present disclosure includes: acquisition unit for acquiring, for each of a plurality of patterns of a material that can be used in a target process, material specification information representing a material specification of the material and physical property information indicating a physical property quantity for each of a plurality of physical properties of a product that can be generated in the target process by using the material; self-organizing map generation unit for generating, by using the physical property information, a self-organizing map on which each node is assigned a position in a map space and a physical property vector indicating a value related to a physical property quantity for each of a plurality of types of physical properties of the product; specification assignment unit for assigning a specification vector indicating a value related to a material specification to each of the nodes by using the material specification information; target node detection unit for acquiring target information indicating a desired physical property of the product and detecting, as a target node, the node to which the physical property vector matching a desired physical property indicated by the target information is assigned; and recommendation data generation unit for generating, by using the specification vector assigned to the target node, recommendation data representing the material specification of the material with which a product having the desired physical property can be generated.

A control method according to the present disclosure is performed by a computer. The control method includes: an acquisition step of acquiring, for each of a plurality of patterns of a material that can be used in a target process, material specification information representing a material specification of the material and physical property information indicating a physical property quantity for each of a plurality of physical properties of a product that can be generated in the target process by using the material; a self-organizing map generation step of generating, by using the physical property information, a self-organizing map on which each node is assigned a position in a map space and a physical property vector indicating a value related to a physical property quantity for each of a plurality of types of physical properties of the product; a specification assignment step of assigning a specification vector indicating a value related to a material specification to each of the nodes by using the material specification information; a target node detection step of acquiring target information indicating a desired physical property of the product and detecting, as a target node, the node to which the physical property vector matching a desired physical property indicated by the target information is assigned; and recommendation data generation step of generating, by using the specification vector assigned to the target node, recommendation data representing the material specification of the material with which a product having the desired physical property can be generated.

A non-transitory computer readable medium according to the present disclosure stores a program for causing a computer to perform a control method according to the present disclosure.

### Advantageous Effects of Invention

According to the present disclosure, a new technique for providing information useful for product development is provided.

### Brief Description of Drawings

Fig. 1 shows an example of an overview of operations performed by a recommendation data generation apparatus according to a first example embodiment;
Fig. 2 is a block diagram showing an example of a functional configuration of the recommendation data generation apparatus according to the first example embodiment;
Fig. 3 is a block diagram showing an example of a hardware configuration of a computer that implements the recommendation data generation apparatus;
Fig. 4 is a flowchart showing an example of a flow of processing performed by the recommendation data generation apparatus according to the first example embodiment;
Fig. 5 shows an example of material specification information in the form of a table;
Fig. 6 shows an example of physical property information in the form of a table;
Fig. 7 shows an example of a structure of a self-organization map on which a specification vector is assigned to each node in the form of a table;
Fig. 8 shows an example of an overview of operations performed by the recommendation data generation apparatus according to the first example embodiment;
Fig. 9 is a block diagram showing an example of a functional configuration of the recommendation data generation apparatus according to the first example embodiment; and
Fig. 10 is a flowchart showing an example of a flow of processing performed by the recommendation data generation apparatus according to the first example embodiment.

### Example Embodiment

An example embodiment according to the present disclosure will be described hereinafter in detail with reference to the drawings. Further, components corresponding to or the same as each other are assigned the same or corresponding numerical numbers (or symbols) throughout the drawings, and redundant descriptions thereof are omitted as appropriate. Further, unless otherwise described, pre-defined information such as predetermined values and thresholds are stored in advance in a storage device or the like accessible from an apparatus that uses these values.

### [First Example Embodiment]

### <Overview>

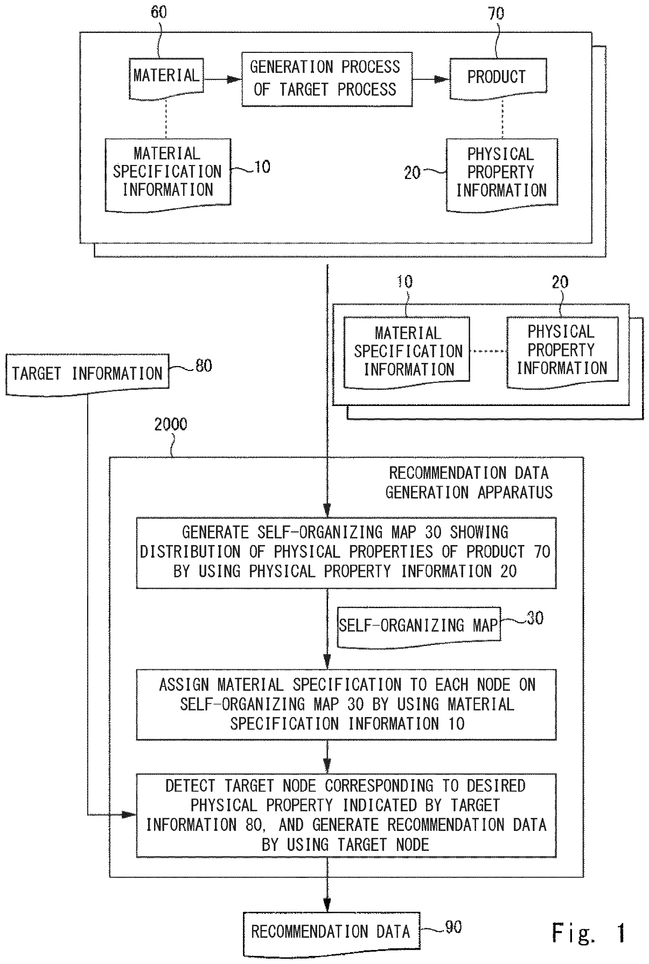

Fig. 1 shows an example of an overview of operations performed by a recommendation data generation apparatus 2000 according to a first example embodiment. Note that Fig. 1 is a diagram merely for facilitating the understanding of the overview of the recommendation data generation apparatus 2000, and the operations performed by the recommendation data generation apparatus 2000 is not limited to those shown in Fig. 1.

The recommendation data generation apparatus 2000 helps, in a specific process (hereinafter also referred to as a target process) in product development, a user or the like search for a material with which a product having desired physical properties can be generated. Note that the target process can be any process through which a product is generated from a material. As means for searching for a material with which a product having desired physical properties can be generated, there is a method in which the generation of a product is simulated or a product is experimentally generated while widely changing its material specifications. The recommendation data generation apparatus 2000 generates data on material specifications that are recommended as those used for such simulations and experimental generation (hereinafter, such data are also referred to as recommendation data). The recommendation data is data on a material specification with which a probability that a product having desired physical properties can be generated is high.

In order to generate such recommendation data, the recommendation data generation apparatus 2000 performs, for example, the following operation. The recommendation data generation apparatus 2000 acquires, for each of a plurality of various patterns of materials 60 (in other words, for materials 60 determined by a plurality of respective patterns of material specifications), material specification information 10 representing a material specification of the material 60 and physical property information 20 indicating physical properties of a product 70 that can be generated in the target process by using the material 60. As described later, a pair of material specification information 10 and physical property information 20 is one that was generated by a simulation(s) that was already performed and/or experimental generation of a product 70.

A pattern of the material 60 is specified by its material specification. In other words, materials 60 having material specifications different from each other are handled as the materials 60 different patterns from each other. On the other hand, materials 60 having the same material specification as each other are handled as the materials 60 of the same pattern as each other.

A material specification is represented by, for example, a type of the material, types of substances constituting the material, a blending ratio of each substance, and a type of processing performed to generate the material. Examples of types of materials include carbon fiber reinforced plastics and stainless steel. For example, assume that the material 60 is a carbon fiber reinforced plastic. In this case, the material specification of the material 60 includes the type of each of one or more carbon fibers that constitute the material 60 (such as polyacrylonitrile fibers and cellulose carbonized fibers), the type of each of one or more resins that constitute the material 60 (such as epoxy and polyether tephthalate), and the blending ratio of those materials. Further, the material specification may also include a type of fiber directional polymerization method, a type of crimping method, and a resin composition.

The physical property information 20 indicates a physical property quantity for each of a plurality of types of physical properties of the product 70. Examples of types of physical properties include incombustibility, heat resistance, elastic modulus, or tenacity. The product 70 is a product that is predicted to be generated or actually generated by processing a material 60 in a generation process of the target process, or an actually generated product.

The recommendation data generation apparatus 2000 generates a self-organizing map 30 showing a distribution of physical properties of the product 70 by using the physical property information 20. The self-organizing map 30 has a plurality of nodes arranged in an m-dimensional map space (m>0).

Each of the nodes on the self-organizing map 30 is assigned multi-dimensional data (hereinafter also referred to as a physical property vector) that represents the magnitude of the physical property quantity for each of a plurality of types of the physical properties. For example, assume that four types of physical properties including incombustibility, heat resistance, elastic modulus, and tenacity, are used. In this case, the physical property vector is a four-dimensional data that represents the magnitude of the physical property quantity for each of these four types of physical properties. In the following description, the number of dimensions of the physical property vector is denoted by n. Note that n is larger than m (n>m). That is, on the self-organizing map 30, the space of the physical property vector is a high-dimensional space while the map space is a low-dimensional space.

The physical property information 20 indicates physical property quantities of at least n types of physical properties. The recommendation data generation apparatus 2000 performs training for the self-organizing map 30 by using physical property quantities of the n types of physical properties indicated by the physical property information 20 and determines a physical property vector to be assigned to each of the nodes, thereby generating a self-organizing map 30.

The recommendation data generation apparatus 2000 assigns a material specification to each of the nodes on the self-organizing map 30 by using the material specification information 10. More specifically, the recommendation data generation apparatus 2000 assigns, to each of the nodes, multi-dimensional data (hereinafter also referred to as a specification vector) representing the value of each of a plurality of types of parameters of the material specification (hereinafter also referred to as specification parameters). In this way, in each of the nodes on the self-organizing map 30, a specification vector and a physical property vector are associated with each other. That is, in each of the nodes, material specifications and physical properties of the product 70 are associated with each other.

Further, the recommendation data generation apparatus 2000 acquires target information 80 representing physical properties of the product 70 desired by the user. The recommendation data generation apparatus 2000 detects a node having a physical property vector that matches desired physical properties indicated by the target information 80 on the self-organizing map 30. In the following description, this node will be referred to as a target node.

The recommendation data generation apparatus 2000 generates recommendation data by using the target node. For example, for a target node or a node located near the target node in the map space, the recommendation data generation apparatus 2000 generates recommendation data indicating the material specification represented by the specification vector assigned to that node.

### <Example of Advantageous Effect>

The recommendation data generation apparatus 2000 generates, by using the physical property information 20, a self-organizing map 30 on which a physical property vector representing physical properties is assigned to each of the nodes. Further, the recommendation data generation apparatus 2000 further assigns, by using the material specification information 10, a specification vector representing a material specification to each of the nodes on the self-organizing map 30. In this way, material specifications and physical properties are associated with each other on the self-organizing map 30. Then, the recommendation data generation apparatus 2000 generates, by using the association, recommendation data 90 representing the material specification of a material 60 with which a probability that a product 70 having desired physical properties can be generated is high. Specifically, the recommendation data generation apparatus 2000 detects a target node having a physical property vector that matches the desired physical properties indicated by the target information 80, and generates recommendation data 90 by using a specification vector associated with the target node or a node located near the target node.

Note that a pair of material specification information 10 and physical property information 20 is one that was generated based on the results of simulations of generation of products 70 or experimental generations thereof that were already performed. Therefore, a pair of material specification information 10 and physical property information 20 can be considered to be knowledge about a relation between material specifications and physical properties that is obtained by simulations or the like that have been performed. According to the recommendation data generation apparatus 2000, it becomes possible to efficiently search for a material 60 with which a product 70 having desired physical properties can be generated (i.e., to search for it in a short time and at a low computational cost) by using such knowledge.

The recommendation data generation apparatus 2000 according to this example embodiment will be described hereinafter in a more detailed manner.

### <Example of Functional Configuration>

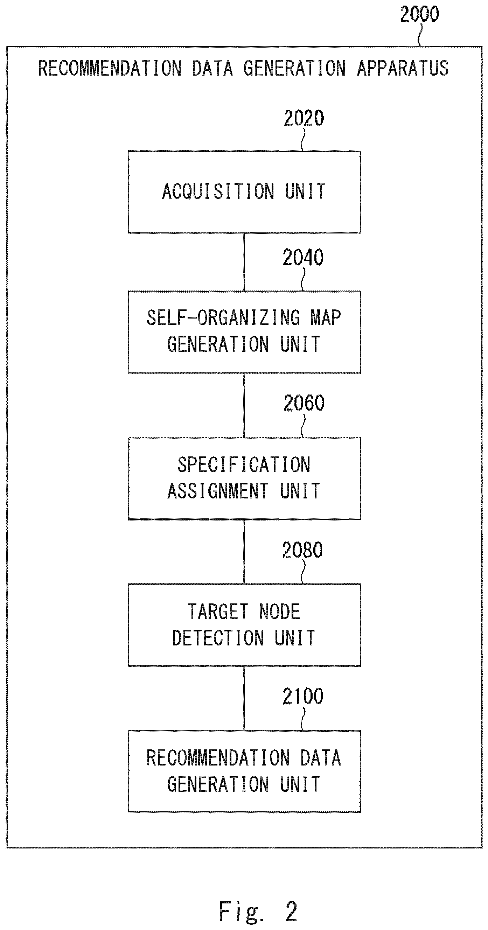

Fig. 2 is a block diagram showing an example of a functional configuration of the recommendation data generation apparatus 2000 according to the first example embodiment. The recommendation data generation apparatus 2000 includes an acquisition unit 2020, a self-organizing map generation unit 2040, a specification assignment unit 2060, a target node detection unit 2080, and a recommendation data generation unit 2100. The acquisition unit 2020 acquires material specification information 10 and physical property information 20 for each of a plurality of various patters of materials 60. The self-organizing map generation unit 2040 generates a self-organizing map 30 by using the physical property information 20. The specification assignment unit 2060 assigns a specification vector to each of the nodes on the self-organizing map 30 by using the material specification information 10. The target node detection unit 2080 acquires target information 80 and detects, as a target node, a node that is assigned a physical property vector matching desired physical properties indicated by the target information 80. The recommendation data generation unit 2100 generates recommendation data 90 by using the target node.

### <Example of Hardware Configuration>

Each of functional components of the recommendation data generation apparatus 2000 can be implemented by hardware that implements the functional component (e.g., a hardwired electronic circuit or the like) or by a combination of hardware and software (e.g., a combination of an electronic circuit and a program for controlling it or the like). A case where each of the functional components of the recommendation data generation apparatus 2000 is implemented by a combination of hardware and software will be further described hereinafter.

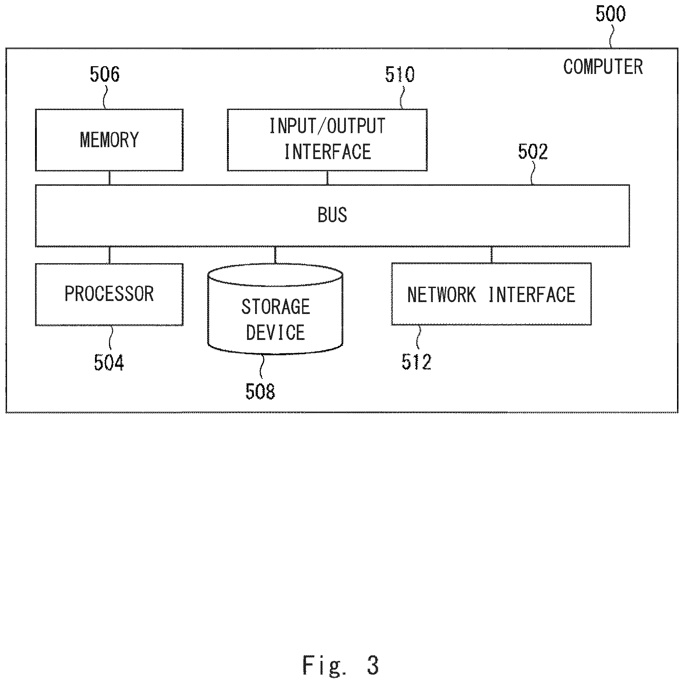

Fig. 3 is a block diagram showing an example of a hardware configuration of a computer 500 that implements the recommendation data generation apparatus 2000. The computer 500 is an arbitrary computer. For example, the computer 500 is a stationary computer such as a server machine or a PC (Personal Computer). Alternatively, for example, the computer 500 is a portable computer such as a smartphone or a tablet-type terminal. The computer 500 may be a special-purpose computer designed to realize the recommendation data generation apparatus 2000, or may be a general-purpose computer.

For example, each of functions of the recommendation data generation apparatus 2000 is implemented by the computer 500 by installing a predetermined application in the computer 500. The aforementioned application is composed of a program for implementing each of the function components of the recommendation data generation apparatus 2000. Note that how to acquire the aforementioned program is arbitrarily determined. For example, the program can be acquired from a storage medium (such as a DVD or a USB memory) in which the program is stored. Alternatively, the program can be acquired, for example, by downloading the program from a server apparatus that manages a storage device in which the program is stored.

The computer 500 includes a bus 502, a processor 504, a memory 506, a storage device 508, an input/output interface 510, and a network interface 512. The bus 502 is a data transmission path through which the processor 504, the memory 506, the storage device 508, the input/output interface 510, and the network interface 512 transmit and receive data to and from each other. However, the method for connecting the processor 504 and the like to each other is not limited to connections through buses.

The processor 504 is any of various types of processors such as a CPU (Central Processing Unit), a GPU (Graphics Processing Unit), or an FPGA (Field-Programmable Gate Array). The memory 506 is a primary storage device implemented by using a RAM (Random Access Memory) or the like. The storage device 508 is a secondary storage device implemented by using a hard disk drive, an SSD (Solid State Drive), a memory card, or a ROM (Read Only Memory).

The input/output interface 510 is an interface for connecting the computer 500 with an input/output device(s). For example, an input device such as a keyboard and an output device such as a display device are connected to the input/output interface 510.

The network interface 512 is an interface for connecting the computer 500 to a network. The network may be a LAN (Local Area Network) or a WAN (Wide Area Network).

In the storage device 508, programs for implementing respective functional components of the recommendation data generation apparatus 2000 (programs for implementing the above-described applications) are stored. The processor 504 implements each of functional components of the recommendation data generation apparatus 2000 by loading the aforementioned program onto the memory 506 and executing the loaded program.

The recommendation data generation apparatus 2000 may be implemented by one computer 500 or by a plurality of computers 500. In the latter case, the configurations of the computers 500 do not need to be identical to each other, but can be different from each other.

### <Flow of Processes>

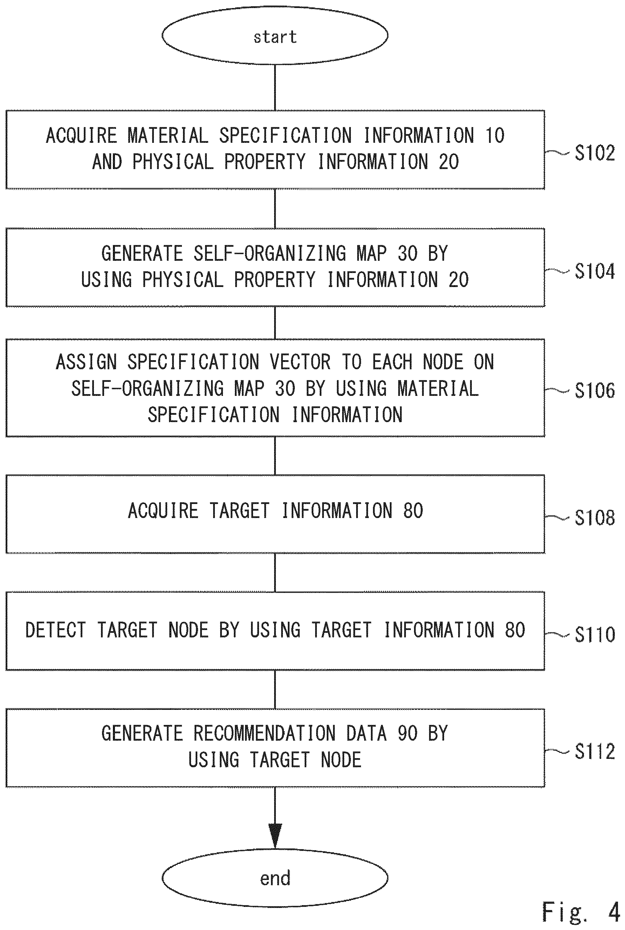

Fig. 4 is a flowchart showing an example of a flow of processing performed by the recommendation data generation apparatus 2000 according to the first example embodiment. The acquisition unit 2020 acquires material specification information 10 and physical property information 20 for each of a plurality of various patterns of materials 60 (S102). The self-organizing map generation unit 2040 generates a self-organizing map 30 by using the physical property information 20 (S104). The specification assignment unit 2060 assigns a specification vector to each of the nodes on the self-organizing map 30 by using the material specification information 10 (S106). The target node detection unit 2080 acquires target information 80 (S108). The target node detection unit 2080 detects a target node by using the target information 80 (S1 10). The recommendation data generation unit 2100 generates recommendation data 90 by using the target node (S112).

### <Acquisition of Material Specification Information 10 and Physical Property Information 20: S102>

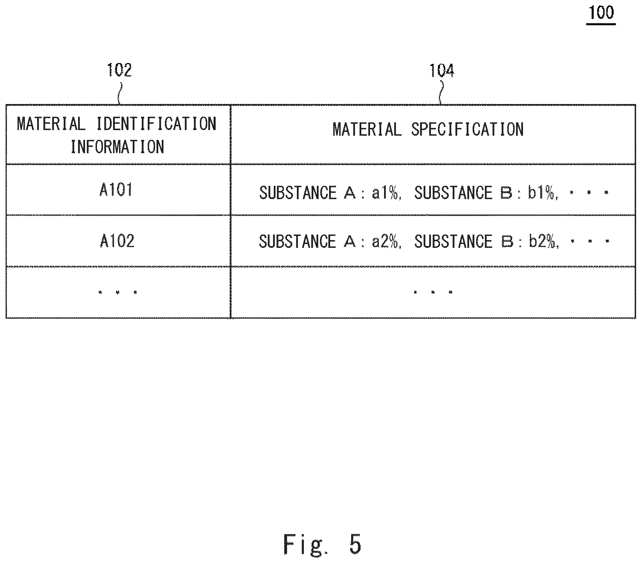

For each of a plurality of various patterns of materials 60 that can be used in the target process, the acquisition unit 2020 acquires material specification information 10 representing a material specification of that material 60 and physical property information 20 for a product 70 that can be generated by using the material 60 (S102). Fig. 5 shows an example of the material specification information 10 in the form of a table. Table 100 in Fig. 5 has a column named material identification information 102 and a column named material specification" 104. The material identification information 102 indicates identification information assigned to a material 60. The material specification 104 indicates a specification of the material 60.

In Fig. 5, the material specification information 10 is represented by one record in Table 100. That is, the material specification information 10 associates the identification information of a material 60 with the material specification of the material 60 having this identification information.

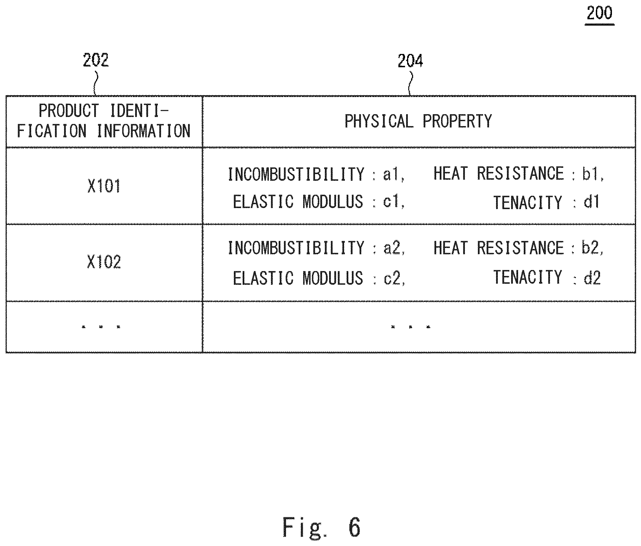

Fig. 6 shows an example of the physical property information 20 in the form of a table. Table 200 in Fig. 6 has a column named product identification information 202 and a column named physical property 204. The product identification information 202 indicates identification information of a product 70. The physical property 204 indicates physical properties of the product 70. In Table 200, each of physical properties of a product 70 is represented by indicating an association "Label indicating Type of Physical Property: Physical Property Quantity of the Physical Property".

In Fig. 6, the physical property information 20 is represented by one record in Table 200. That is, the physical property information 20 associates the identification information of a product 70 with the physical properties of the product 70 having that identification information.

The acquisition unit 2020 acquires a plurality of pairs of the material specification information 10 and the physical property information 20. There are various methods by which the acquisition unit 2020 acquires the pairs of the material specification information 10 and the physical property information 20. For example, pairs of the material specification information 10 and the physical property information 20 are stored in advance in an arbitrary storage device accessible from the recommendation data generation apparatus 2000. The acquisition unit 2020 acquires a pair of the material specification information 10 and the physical property information 20 by accessing this storage device. Alternative, for example, the acquisition unit 2020 may acquire a pair of the material specification information 10 and the physical property information 20 by receiving an input from a user for entering the pair of the material specification information 10 and the physical property information 20. Alternatively, for example, the acquisition unit 2020 may acquire a pair of the material specification information 10 and the physical property information 20 by receiving the pair of the material specification information 10 and the physical property information 20 transmitted from other apparatuses.

Note that there are various methods for generating a pair of the material specification information 10 and the physical property information 20. For example, a pair of the material specification information 10 and the physical property information 20 is generated by performing a simulation of the generation of a product 70. Specifically, by performing a simulation given an input of a specific material specification, physical property information 20 that indicates a predicted physical property quantity of each physical property of a product 70 is generated. Then, a pair of the generated physical property information 20 and the material specification information 10 indicating the material specification given as the input is obtained. Note that an existing technique can be used for the above-described technique in which a material specification is acquired as an input and a simulation for outputting predicted data of physical properties of a product that is generated in a specific process using a material specified by the acquired material specifications is performed.

Alternatively, for example, a pair of the material specification information 10 and the physical property information 20 can be generated by actually producing a product 70. Specifically, a product 70 is experimentally generated by using a material 60 represented by a specific material specification in the target process. Further, physical property information 20 is generated by measuring a physical property quantity of each of physical properties of the generated product 70. As a result, a pair of the generated physical property information 20 and the material specification information 10 representing the used material 60 is obtained.

Note that a plurality of pieces of physical property information 20 acquired by the acquisition unit 2020 may include those that express data in different ways from each other. For example, it is conceivable that different labels are used for physical properties that are essentially the same as each other. Further, it is conceivable that physical property quantities of the same physical property are expressed in units different from each other. In such a case, it is preferred that the acquisition unit 2020 unifies the ways of expressing data, for example, by unifying labels or performing inter-unit conversion. It is conceivable that such a situation in which the ways of expressing data of pieces of physical property information 20 are different from each other could occur, for example, when pieces of physical property information 20 generated by using a simulation and pieces of physical property information 20 generated by experimentally generating a product 70 are both acquired. Note that it is preferred that the unification of ways of expressing data is also carried out for the material specification information 10 in a similar manner.

### <Generation of Self-Organizing Map 30: S104>

The self-organizing map generation unit 2040 generates a self-organizing map 30 by using the physical property information 20 (S104). The self-organizing map 30 has a plurality of nodes arranged in an m-dimensional map space. The number of dimensions of the map space may be determined in advance or designated by a user.

Each of the nodes on the self-organizing map 30 is assigned an n-dimensional physical property vector. The assignment of a physical property vector to each node is carried out through the training of the self-organizing map 30. The training of the self-organizing map 30 can be carried out by inputting n-dimensional training data to be used for the training into the self-organizing map 30. Note that an existing method can be used as an actual method for training the self-organizing map by using training data.

For example, the self-organizing map generation unit 2040 initializes the self-organizing map 30 by an arbitrary method. As the initialization method, for example, a method to initialize a physical property vector of each node to a random value can be adopted. The self-organizing map generation unit 2040 extracts a physical property quantity for each of the n-types of the physical properties from each of the acquired pieces of the physical property information 20 to generate n-dimensional physical property vectors. The self-organizing map generation unit 2040 performs the training of the self-organizing map 30 using each of the physical property vectors as training data, thereby generating the self-organizing map 30. As a result, a physical property vector of each node on the self-organizing map 30 becomes n-dimensional data indicating a value for each of the physical property quantities of n types of the physical properties.

The physical property vector obtained from the physical property information 20 may indicate each of physical property quantities of the n types of the physical properties represented by the physical property information 20 as it is, or may indicate a value that is obtained by converting each of physical property quantities with a predetermined method (e.g., normalization, standardization, or the like).

Note that the number of physical properties represented by the physical property information 20 may be greater than n. In this case, some of data represented by the physical property information 20 are used to generate the self-organizing map 30. Note that which types of physical properties represented by the physical property information 20 are used to generate the self-organizing map 30 may be determined in advance or designated by a user.

### <Assignment of Specification Vector: S106>

The specification assignment unit 2060 assigns a specification vector to each node on the self-organizing map 30 by using the material specification information 10 (S106). Note that the specification vector obtained from the material specification information 10 may indicate values of all the specification parameters indicated by the material specification information 10 or values of some of all the specification parameters. That is, when the number of dimensions of the specification vector is represented by k, the value of k may be equal to the number of the specification parameters indicated by the material specification information 10 or smaller than the number of the specification parameters indicated by the material specification information 10.

For example, assume that the material specification information 10 indicates both parameters that have continuous values (e.g., a blending ratio of a substance) and parameters that do not have continuous values (e.g., a type of processing or the like). In this case, the specification vector is generated by parameters that have continuous values.

Note that which of the parameters indicated by the material specification information 10 are used to generate the specification vector may be determined in advance or designated by a user. Further, the specification vector may indicate the value of a parameter indicated by the material specification information 10 as it is, or may indicate a value that is obtained by converting the value of a respective parameter with a predetermined method (e.g., normalization, standardization, or the like).

In order to determine the assignment of a specification vector to each node, for example, the specification assignment unit 2060 assigns each of pieces of the material specification information 10 to a node. The physical property information 20 corresponding to the material specification information 10 is used for the assignment of the material specification information 10 to the node. That is, the specification assignment unit 2060 determines a node having a physical property vector that is most similar to a physical property vector obtained from the physical property information 20 corresponding to the material specification information 10. The specification assignment unit 2060 assigns the material specification information 10 to the determined node. The specification assignment unit 2060 assigns the specification vector obtained from the material specification information 10 to the node to which that material specification information 10 is assigned.

Note that as a result of the above-described process, there may be a node to which no material specification information 10 is assigned. Therefore, for example, the specification assignment unit 2060 obtains, by estimation, a specification vector to be assigned to a node to which no material specification information 10 has been assigned. That is, the specification assignment unit 2060 estimates a distribution of specification vectors based on the specification vectors of the respective nodes to which the material specification information 10 has been assigned and the arrangement of these nodes in the map space. Then, the specification assignment unit 2060 also assigns a specification vector to the node to which no material specification information 10 has been assigned by using the estimated distribution.

There are various methods for estimating a distribution of specification vectors. For example, the specification assignment unit 2060 estimates a distribution of specification vectors by using an arbitrary interpolation process such as linear interpolation or spline interpolation. Alternatively, for example, the specification assignment unit 2060 may estimate a distribution of specification vectors by sparse estimation. Note that when a distribution of specification vectors is estimated, the accuracy of the estimation may be improved by further applying Bayesian estimation.

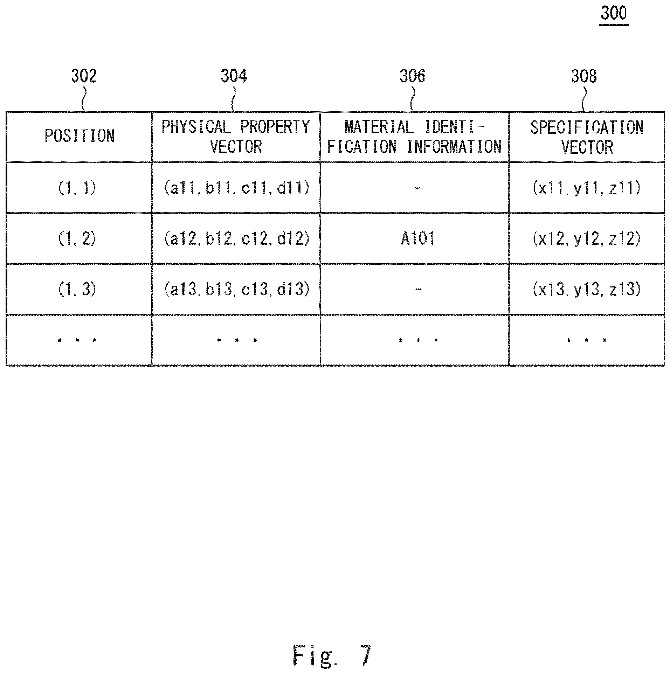

Fig. 7 shows an example of a structure of a self-organizing map 30 on which a specification vector is assigned to each node in the form of a table. Table 300 in Fig. 7 has four columns: position 302, physical property vector 304, material identification information 306, and specification vector 308. Table 300 has one record for one node.

The position 302 indicates coordinates of a node in the map space. In the example shown in Fig. 7, m is two (m=2), and x-coordinate and y-coordinate are assigned to a node. The physical property vector 304 shows a n-dimensional physical property vector assigned to the node. In the example shown in Fig. 7, n is four (n=4). For a node to which material specification information 10 is assigned, the material identification information 306 shows identification information of a material 60 indicated by the material specification information 10 assigned to that node. In a record of a node to which no material specification information 10 is assigned, the material identification information 306 shows "-". The specification vector 308 shows k-dimensional specification vectors assigned to the node. In the example shown in Fig. 7, k is three (k=3).

### <Acquisition of Target Information 80: S108>

The target node detection unit 2080 acquires target information 80 (S 108). The target information 80 represents physical properties of a product 70 desired by the user of the recommendation data generation apparatus 2000. For example, the target information 80 indicates a desired value of a physical property quantity for each of at least one type of physical property of the product 70. Alternatively, for example, the target information 80 may indicate a desired range of the physical property quantity for each of at least one type of physical property of the product 70.

The method by which the target node detection unit 2080 acquires target information 80 is arbitrarily determined. For example, the target information 80 is stored in advance in an arbitrary storage device accessible from the recommendation data generation apparatus 2000. The target node detection unit 2080 acquires the target information 80 by accessing this storage device. Alternatively, for example, the target node detection unit 2080 receives an input operation for designating desired physical properties from a user and acquires target information 80 as a result of the input. Alternatively, for example, the target node detection unit 2080 may acquire target information 80 by receiving target information 80 transmitted from other apparatuses.

### <Detection of Target Node: S110>

The target node detection unit 2080 detects a target node by using the target information 80 (S110). To do so, the target node detection unit 2080 determines, for each node on the self-organizing map 30, whether or not a physical property vector assigned to that node matches the desired physical properties indicated by the target information 80.

For example, assume that the target information 80 indicates a desired value for each of at least one type of physical property. In this case, when the physical property vector of a given node indicates, for each of all the physical properties indicated by the target information 80, a value that is equal to the desired value of that physical property, that node is detected as the target node. Note that in the case where the physical property vector indicates values that are obtained by, for example, normalizing physical property quantities, the fact that "the physical property vector indicates values that are equal to desired values" means that when the values indicated by the physical property vector are converted into physical property quantities, the resultant values are equal to their desired values.

Alternatively, for example, assume that the target information 80 indicates a desired range for each of at least one type of physical property. In this case, when the physical property vector of a given node indicates, for each of all the physical properties indicated by the target information 80, a value that is within the desired range for that physical property, that node is detected as the target node. Note that in the case where the physical property vector indicates values that are obtained by, for example, normalizing physical property quantities, the fact that "the physical property vector indicates values that are within desired ranges" means that when the values indicated by the physical property vector are converted into physical property quantities, the resultant values are within their desired ranges.

Note that on the self-organizing map 30, there may be a plurality of physical property vectors which match the same desired physical property. Therefore, the target node detection unit 2080 may detect each of a plurality of nodes as the target node.

### <Generation of Recommendation Data: S112>

The recommendation data generation unit 2100 generates recommendation data 90 by using the target node (S112). Specifically, the recommendation data generation unit 2100 generates recommendation data 90 by using a specification vector assigned to the target node. For example, assume that a specification vector indicates values of a plurality of specification parameters as they are. In this case, the recommendation data generation unit 2100 generates recommendation data 90 representing the material specification specified by the values themselves indicated by the specification vector. In contrast, assume that a specification vector does not indicate values of a plurality of specification parameters as they are, but does indicate values obtained by converting them by a certain method such as normalization. In this case, the recommendation data generation unit 2100 converts values indicated by the specification vector into values of specifications parameters, and generates recommendation data 90 by using the converted values.

Note that the recommendation data generation unit 2100 may add, into the recommendation data 90, a value of a specification parameter that is not included in the specification vector. For example, assume that the specification vector does not indicate any value regarding the type of material. In this case, the recommendation data generation unit 2100 adds a value representing the type of material in the recommendation data 90 generated from the specification vector. In this case, the type of material to be added may be determined in advance, designated by a user, or specified by using the material specification information 10. For example, assume that all of a plurality of pieces of material specification information 10 acquired by the recommendation data generation apparatus 2000 indicate the same type of material. In this case, the recommendation data generation unit 2100 adds a value indicating that same type of material as that indicated by the pieces of material specification information 10 in the recommendation data 90.

The recommendation data generation unit 2100 may generate a plurality of recommendation data 90. In this case, for example, the recommendation data generation unit 2100 selects at least one other node located near the target node in the map space, and generates, for each of the selected nodes, recommendation data 90 from the specification vector assigned to that node. The method for generating recommendation data 90 from each node is similar to the method for generating recommendation data 90 from the target node.

There are various methods for selecting a node that is used to generate recommendation data 90. For example, the recommendation data generation unit 2100 selects at least one node from a sub-area including the target node in the map space. For example, a sub-area is an area whose center is at the target node and in which a distance from the target node is equal to or shorter than a predetermined threshold. Note that regarding the type of distance, any type of distance, such as a Euclidean distance or a Manhattan distance, can be used.

The sub-area may be defined by performing clustering on the map space. Specifically, the recommendation data generation unit 2100 divides the map space into a plurality of clusters based on the specification vectors assigned to each node. In this way, nodes to which specification vectors similar to each other are assigned are classified into the same cluster. The recommendation data generation unit 2100 uses the cluster to which the target node belongs as the aforementioned predetermined range. That is, the recommendation data generation unit 2100 generates recommendation data 90 from each of the nodes belonging to the cluster same as that the target node belongs to. Note that an existing clustering algorithm such as a k-means method can be used as for the technique for performing clustering on the nodes based on the vectors associated with the respective nodes.

When the recommendation data generation unit 2100 selects a plurality of nodes from the above-described sub-area, the order of the selections of the nodes is arbitrarily determined. For example, the recommendation data generation unit 2100 selects the nodes from the sub-area in a random order. Alternatively, for example, the recommendation data generation unit 2100 selects the nodes in an ascending order of the distance from the target node. Note that when there is a plurality of nodes each of whose distance from the target node is equal to each other, the recommendation data generation unit 2100 may select these nodes in a random order or according to a predetermined rule (e.g., in an ascending order of identifiers, in a clockwise order in the map space, or the like).

The recommendation data generation unit 2100 may use all the nodes included in the sub-area or may use some of them. In the latter case, for example, the recommendation data generation unit 2100 selects a predetermined number of nodes. The predetermined number may be determined in advance or designated by a user.

Alternatively, for example, the recommendation data generation unit 2100 may repeat the selection of a node while recommendation data 90 is requested. For example, every time the recommendation data generation unit 2100 generates recommendation data 90, it acquires input data indicating whether or not additional recommendation data 90 is required. When the input data indicates that additional recommendation data 90 is required, the recommendation data generation unit 2100 selects a node that has not been selected yet and generates recommendation data 90 by using the selected node. On the other hand, when the input data indicates that recommendation data 90 is no longer required, the recommendation data generation unit 2100 finishes the selection of a node.

The above-described input data is input, for example, by a user. In this case, for example, every time the recommendation data generation apparatus 2000 generates recommendation data 90, it displays, on a display device of a terminal that the user can operate, an input screen through which the recommendation data generation apparatus 2000 receives an input as to whether or not recommendation data 90 needs to be further generated. The recommendation data generation apparatus 2000 acquires the result of the input by the user as the above-described input data.

Alternatively, for example, the above-described input data may be transmitted from a simulator that uses recommendation data 90. In this case, the recommendation data generation apparatus 2000 and the simulator operate in cooperation with each other. For example, the recommendation data generation apparatus 2000 provides recommendation data 90 that the recommendation data generation apparatus 2000 has generated to the simulator. The simulator uses the provided recommendation data 90 to perform the simulation of the generation of a product 70. After that, when an additional simulation needs to be performed, the simulator transmits an input requesting the next recommendation data 90 (an input indicating that additional recommendation data 90 is required as described above) to the recommendation data generation apparatus 2000. Upon receiving the above-described request, the recommendation data generation apparatus 2000 further selects a node and generates recommendation data 90.

The method for selecting a node is not limited to the method in which a node is selected from a sub-area. For example, the recommendation data generation unit 2100 may select a predetermined number of nodes in an ascending order of the distance from the target node.

### <Output of Recommendation Data 90>

The recommendation data 90 can be output in various manners. For example, the recommendation data generation apparatus 2000 puts the recommendation data 90 in an arbitrary storage device accessible from the recommendation data generation apparatus 2000. Alternatively, for example, the recommendation data generation apparatus 2000 displays the recommendation data 90 on an arbitrary display device controllable from the recommendation data generation apparatus 2000. Alternatively, for example, the recommendation data generation apparatus 2000 transmits the recommendation data 90 to an arbitrary apparatus (e.g., the above-described simulator) that is connected to the recommendation data generation apparatus 2000 so that they can communicate with each other.

### <Example Usage of Recommendation Data 90>

For example, the recommendation data 90 can be used to simulate the generation of a product 70 or to experimentally generate a product 70. Note that the recommendation data 90 may be referred to by an operator who performs the aforementioned simulation or who experimentally generates a product or the like, or may be referred to by a simulator that performs a simulation. In the former case, the operator may perform the simulation by entering the recommendation data 90 into the simulator, or experimentally generate a product 70 by preparing a material 60 specified by material specifications indicated by the recommendation data 90. The latter case will be described hereinafter as a second example embodiment.

### [Second Example Embodiment]

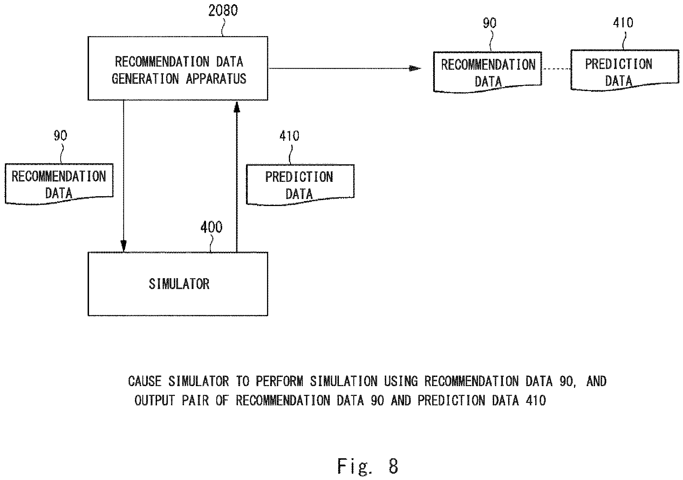

Fig. 8 shows an example of an overview of operations performed by a recommendation data generation apparatus 2000 according to a second example embodiment. Note that Fig. 8 is a diagram for facilitating the understanding of the overview of the recommendation data generation apparatus 2000, and the operations performed by the recommendation data generation apparatus 2000 is not limited to those shown in Fig. 8.

The recommendation data generation apparatus 2000 causes a simulator 400 to perform a simulation by inputting recommendation data 90 into the simulator 400. The simulator 400 simulates a generation process of a target process. Specifically, the simulator 400 acquires input data representing a material specification and generates prediction data 410 representing predicted physical properties of the product 70, regarding the product 70 that is generated by using the material 60 specified by the input data. Note that as the simulator 400, an arbitrary existing simulator can be used as long as it can acquire input data representing a material specification, perform a simulation by using the input data, and output prediction data on physical properties of a product. Further, the simulator 400 may be implemented by the computer that implements the recommendation data generation apparatus 2000, or may be implemented by another computer(s).

The recommendation data generation apparatus 2000 acquires the prediction data 410 generated by the simulator 400 and outputs a pair of the recommendation data 90 and the prediction data 410. In this way, a pair of the recommendation data 90 and the prediction data 410 that has been obtained by inputting the recommendation data 90 into the simulator 400 is output.

It can be said that this pair is one that corresponds to the above-described pair of the material specification information 10 and the physical property information 20. Therefore, by using the recommendation data generation apparatus 2000, it is possible to improve, based on the results of simulations for the generation of a product 70 that were already performed or the results of experiments therefor, the efficiency of simulations that will be subsequently performed. For example, for a certain number of times of simulations that are performed first, material specifications with which simulations experimental generations are performed are determined based on past knowledge or determined in a random manner. After that, their results are input into the recommendation data generation apparatus 2000, so that recommendation data 90 representing a material specification with which a probability that a product 70 having desired physical properties can be generated is high is obtained. Therefore, since simulations can be performed while focusing on material specifications with which the probability that a product 70 having desired physical properties can be generated is high, the simulations can be efficiently performed.

The pair of recommendation data 90 and prediction data 410 can be output in various manners. For example, the recommendation data generation apparatus 2000 puts the aforementioned pair in an arbitrary storage device accessible from the recommendation data generation apparatus 2000. Alternatively, for example, the recommendation data generation apparatus 2000 displays the aforementioned pair on an arbitrary display device controllable from the recommendation data generation apparatus 2000. Alternatively, for example, the recommendation data generation apparatus 2000 transmits the aforementioned pair to an arbitrary apparatus that is connected to the recommendation data generation apparatus 2000 so that they can communicate with each other.

Note that the recommendation data generation apparatus 2000 may cause the simulator 400 to perform a simulation using the recommendation data 90 every time the recommendation data 90 is generated, or it may generate a plurality of recommendation data 90 and then cause the simulator 400 to perform the simulation for each of the plurality of recommendation data 90 in turn.

### <Example of Functional Configuration>

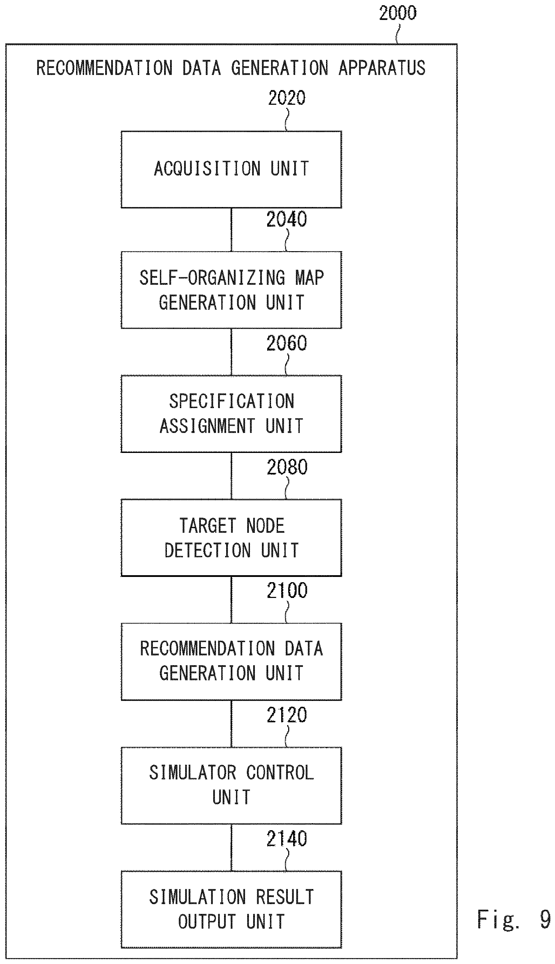

Fig. 9 is a block diagram showing an example of a functional configuration of the recommendation data generation apparatus 2000 according to the second example embodiment. The recommendation data generation apparatus 2000 according to the second example embodiment further includes a simulator control unit 2120 and a simulation result output unit 2140. The simulator control unit 2120 causes, by inputting recommendation data 90 into the simulator 400, the simulator 400 to perform a simulation of the generation of a product 70 using the material 60 specified by the material specification represented by the recommendation data 90. The simulation result output unit 2140 acquires prediction data 410 output from the simulator 400 and outputs a pair of the recommendation data 90 and the prediction data 410.

### <Example of Hardware Configuration>

Similarly to the recommendation data generation apparatus 2000 according to the first example embodiment, the recommendation data generation apparatus 2000 according to the second example embodiment has, for example, the hardware configuration shown in Fig. 3. However, a program for implementing each of the functional components of the recommendation data generation apparatus 2000 according to the second example embodiment is stored in the storage device 508 in the second example embodiment.

### <Flow of Processes>

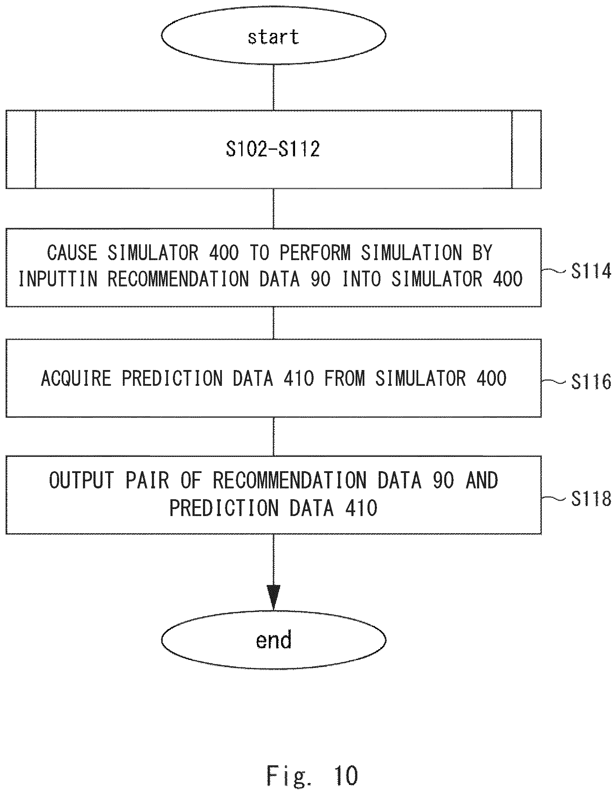

Fig. 10 is a flowchart showing an example of a flow of processing performed by the recommendation data generation apparatus 2000 according to the second example embodiment. Note that steps S102 to S112 are the same as those shown in Fig. 4, and hence are not shown in Fig. 10. In a step S 114, the simulator control unit 2120 inputs recommendation data 90 into the simulator 400 and thereby causes the simulator 400 to perform a simulation. The simulation result output unit 2140 acquires prediction data 410 from the simulator 400 (S116). The simulation result output unit 2140 outputs a pair of the recommendation data 90 and the prediction data 410 (S118).

Although the present invention is described above with reference to example embodiments, the present invention is not limited to the above-described example embodiments. Various modifications that can be understood by those skilled in the art can be made to the configuration and details of the present invention within the scope of the invention.

Note that, in the above-described examples, the program can be stored and provided to a computer using any type of non-transitory computer readable media. Non-transitory computer readable media include any type of tangible storage media. Examples of non-transitory computer readable media include magnetic storage media (such as floppy disks, magnetic tapes, hard disk drives, etc.), optical magnetic storage media (e.g., magneto-optical disks), CD-ROM, CD-R, CD-R/W, and semiconductor memories (such as mask ROM, PROM (Programmable ROM), EPROM (Erasable PROM), flash ROM, RAM, etc.). Further, the program may be provided to a computer using any type of transitory computer readable media. Examples of transitory computer readable media include electric signals, optical signals, and electromagnetic waves. Transitory computer readable media can provide the program to a computer via a wired communication line (e.g., electric wires, and optical fibers) or a wireless communication line.

The whole or part of the example embodiments disclosed above can be described as, but not limited to, the following supplementary notes.

### (Supplementary Note 1)

A recommendation data generation apparatus comprising:
acquisition means for acquiring, for each of a plurality of patterns of a material that can be used in a target process, material specification information representing a material specification of the material and physical property information indicating a physical property quantity for each of a plurality of physical properties of a product that can be generated in the target process by using the material;
self-organizing map generation means for generating, by using the physical property information, a self-organizing map on which each node is assigned a position in a map space and a physical property vector indicating a value related to a physical property quantity for each of a plurality of types of physical properties of the product;
specification assignment means for assigning a specification vector indicating a value related to a material specification to each of the nodes by using the material specification information;
target node detection means for acquiring target information indicating a desired physical property of the product and detecting, as a target node, the node to which the physical property vector matching a desired physical property indicated by the target information is assigned; and
recommendation data generation means for generating, by using the specification vector assigned to the target node, recommendation data representing the material specification of the material with which a product having the desired physical property can be generated.

### (Supplementary Note 2)

The recommendation data generation apparatus according to Supplementary note 1,
wherein the recommendation data generation means selects at least one of the nodes from a sub-area in which the target node is included in the map space, and further generates, for each of the selected nodes, the recommendation data by using the specification vector assigned to that node.

### (Supplementary Note 3)

The recommendation data generation apparatus according to Supplementary note 2,
wherein the recommendation data generation means uses, as the sub-area, an area whose center is at the target node and in which a distance from the target node is equal to or shorter than a threshold.

### (Supplementary Note 4)

The recommendation data generation apparatus according to Supplementary note 2,
wherein the recommendation data generation means performs:
dividing the map space into a plurality of clusters based on the specification vector assigned to each of the nodes; and
using the cluster to which the target node belongs as the sub-area.

### (Supplementary Note 5)

The recommendation data generation apparatus according to Supplementary note 1,
wherein the recommendation data generation means further generates the recommendation data for each of a predetermined number of nodes in an ascending order of the distance from the target node in the map space by using the specification vector assigned to the node.

### (Supplementary Note 6)

The recommendation data generation apparatus according to any one of Supplementary notes 1 to 5, further comprising:
simulator control means for causing, for the product that can be generated by using a material specified by the material specification being input, a simulator to perform a simulation taking the material specification represented by the recommendation data as an input, the simulator being configured to generate prediction data of a physical property of the product; and
simulation result output means for acquiring the prediction data generated by the simulator and outputting the prediction data and the recommendation data.

### (Supplementary Note 7)

A control method performed by a computer, comprising:
an acquisition step of acquiring, for each of a plurality of patterns of a material that can be used in a target process, material specification information representing a material specification of the material and physical property information indicating a physical property quantity for each of a plurality of physical properties of a product that can be generated in the target process by using the material;
a self-organizing map generation step of generating, by using the physical property information, a self-organizing map on which each node is assigned a position in a map space and a physical property vector indicating a value related to a physical property quantity for each of a plurality of types of physical properties of the product;
a specification assignment step of assigning a specification vector indicating a value related to a material specification to each of the nodes by using the material specification information;
a target node detection step of acquiring target information indicating a desired physical property of the product and detecting, as a target node, the node to which the physical property vector matching a desired physical property indicated by the target information is assigned; and
recommendation data generation step of generating, by using the specification vector assigned to the target node, recommendation data representing the material specification of the material with which a product having the desired physical property can be generated.

### (Supplementary Note 8)

The control method according to Supplementary note 7,
wherein in the recommendation data generation step, selecting at least one of the nodes from a sub-area in which the target node is included in the map space, and further generating the recommendation data for each of the selected nodes by using the specification vector assigned to that node.

### (Supplementary Note 9)

The control method according to Supplementary note 8,
wherein in the recommendation data generation step, using, as the sub-area, an area whose center is at the target node and in which a distance from the target node is equal to or shorter than a threshold.

### (Supplementary Note 10)

The control method according to Supplementary note 8,
wherein in the recommendation data generation means, performing:
dividing the map space into a plurality of clusters based on the specification vectors assigned to the respective nodes; and
using the cluster to which the target node belongs as the sub-area.

### (Supplementary Note 11)

The control method according to Supplementary note 7,
wherein in the recommendation data generation step, further generating the recommendation data for each of a predetermined number of nodes in an ascending order of the distance from the target node in the map space by using the specification vector assigned to the node.

### (Supplementary Note 12)

The control method according to any one of Supplementary notes 7 to 11, further comprising:
a simulator control step of causing, for the product that can be generated by using a material specified by the material specification being input, a simulator to perform a simulation taking the material specification represented by the recommendation data as an input, the simulator being configured to generate prediction data of a physical property of the product; and
a simulation result output step of acquiring the prediction data generated by the simulator and outputting the prediction data and the recommendation data.

### (Supplementary Note 13)

A non-transitory computer readable medium for causing a computer to perform:
an acquisition step of acquiring, for each of a plurality of patterns of a material that can be used in a target process, material specification information representing a material specification of the material and physical property information indicating a physical property quantity for each of a plurality of physical properties of a product that can be generated in the target process by using the material;
a self-organizing map generation step of generating, by using the physical property information, a self-organizing map on which each node is assigned a position in a map space and a physical property vector indicating a value related to a physical property quantity for each of a plurality of types of physical properties of the product;
a specification assignment step of assigning a specification vector indicating a value related to a material specification to each of the nodes by using the material specification information;
a target node detection step of acquiring target information indicating a desired physical property of the product and detecting, as a target node, the node to which the physical property vector matching a desired physical property indicated by the target information is assigned; and
recommendation data generation step of generating, by using the specification vector assigned to the target node, recommendation data representing the material specification of the material with which a product having the desired physical property can be generated.

### (Supplementary Note 14)

The non-transitory computer readable medium according to Supplementary note 13,
wherein in the recommendation data generation step, selecting at least one of the nodes from a sub-area in which the target node is included in the map space, and further generating the recommendation data for each of the selected nodes by using the specification vector assigned to that node.

### (Supplementary Note 15)

The non-transitory computer readable medium according to Supplementary note 14,
wherein in the recommendation data generation step, using, as the sub-area, an area whose center is at the target node and in which a distance from the target node is equal to or shorter than a threshold.

### (Supplementary Note 16)

The non-transitory computer readable medium according to Supplementary note 14,
wherein in the recommendation data generation means, performing:
dividing the map space into a plurality of clusters based on the specification vectors assigned to the respective nodes; and
using the cluster to which the target node belongs as the sub-area.

### (Supplementary Note 17)

The non-transitory computer readable medium according to Supplementary note 13,
wherein in the recommendation data generation step, further generating the recommendation data for each of a predetermined number of nodes in an ascending order of the distance from the target node in the map space by using the specification vector assigned to the node.

### (Supplementary Note 18)

The non-transitory computer readable medium according to any one of Supplementary notes 13 to 17, further comprising:
a simulator control step of causing, for the product that can be generated by using a material specified by the material specification being input, a simulator to perform a simulation taking the material specification represented by the recommendation data as an input, the simulator being configured to generate prediction data of a physical property of the product; and
a simulation result output step of acquiring the prediction data generated by the simulator and outputting the prediction data and the recommendation data.

This application is based upon and claims the benefit of priority from Japanese patent application No. 2021-044485, filed on March 18, 2021, the disclosure of which is incorporated herein in its entirety by reference.

### Reference Signs List

- 10: MATERIAL SPECIFICATION INFORMATION
- 20: PHYSICAL PROPERTY INFORMATION
- 30: SELF-ORGANIZATION MAP
- 60: MATERIAL
- 70: PRODUCT
- 80: TARGET INFORMATION
- 90: RECOMMENDATION DATA
- 100: TABLE
- 102: MATERIAL IDENTIFICATION INFORMATION
- 104: MATERIAL SPECIFICATION
- 200: TABLE
- 202: PRODUCT IDENTIFICATION INFORMATION
- 204: PHYSICAL PROPERTY
- 300: TABLE
- 302: POSITION
- 304: PHYSICAL PROPERTY VECTOR
- 306: MATERIAL IDENTIFICATION INFORMATION
- 308: SPECIFICATION VECTOR
- 400: SIMULATOR
- 410: PREDICTION DATA
- 500: COMPUTER
- 502: BUS
- 504: PROCESSOR
- 506: MEMORY
- 508: STORAGE DEVICE
- 510: INPUT/OUTPUT INTERFACE
- 512: NETWORK INTERFACE
- 2000: RECOMMENDATION DATA GENERATION APPARATUS
- 2020: ACQUISITION UNIT
- 2040: SELF-ORGANIZING MAP GENERATION UNIT
- 2060: SPECIFICATION ASSIGNMENT UNIT
- 2080: TARGET NODE DETECTION UNIT
- 2100: RECOMMENDATION DATA GENERATION UNIT
- 2120: SIMULATOR CONTROL UNIT
- 2140: SIMULATION RESULT OUTPUT UNIT

## Claims

1. A recommendation data generation apparatus comprising:
acquisition means for acquiring, for each of a plurality of patterns of a material that can be used in a target process, material specification information representing a material specification of the material and physical property information indicating a physical property quantity for each of a plurality of physical properties of a product that can be generated in the target process by using the material;
self-organizing map generation means for generating, by using the physical property information, a self-organizing map on which each node is assigned a position in a map space and a physical property vector indicating a value related to a physical property quantity for each of a plurality of types of physical properties of the product;
specification assignment means for assigning a specification vector indicating a value related to a material specification to each of the nodes by using the material specification information;
target node detection means for acquiring target information indicating a desired physical property of the product and detecting, as a target node, the node to which the physical property vector matching a desired physical property indicated by the target information is assigned; and
recommendation data generation means for generating, by using the specification vector assigned to the target node, recommendation data representing the material specification of the material with which a product having the desired physical property can be generated.

2. The recommendation data generation apparatus according to claim 1,
wherein the recommendation data generation means selects at least one of the nodes from a sub-area in which the target node is included in the map space, and further generates, for each of the selected nodes, the recommendation data by using the specification vector assigned to that node.

3. The recommendation data generation apparatus according to claim 2,
wherein the recommendation data generation means uses, as the sub-area, an area whose center is at the target node and in which a distance from the target node is equal to or shorter than a threshold.

4. The recommendation data generation apparatus according to claim 2,
wherein the recommendation data generation means performs:
dividing the map space into a plurality of clusters based on the specification vector assigned to each of the nodes; and
using the cluster to which the target node belongs as the sub-area.

5. The recommendation data generation apparatus according to claim 1,
wherein the recommendation data generation means further generates the recommendation data for each of a predetermined number of nodes in an ascending order of the distance from the target node in the map space by using the specification vector assigned to the node.

6. The recommendation data generation apparatus according to any one of claims 1 to 5, further comprising:
simulator control means for causing, for the product that can be generated by using a material specified by the material specification being input, a simulator to perform a simulation taking the material specification represented by the recommendation data as an input, the simulator being configured to generate prediction data of a physical property of the product; and
simulation result output means for acquiring the prediction data generated by the simulator and outputting the prediction data and the recommendation data.

7. A control method performed by a computer, comprising:
an acquisition step of acquiring, for each of a plurality of patterns of a material that can be used in a target process, material specification information representing a material specification of the material and physical property information indicating a physical property quantity for each of a plurality of physical properties of a product that can be generated in the target process by using the material;
a self-organizing map generation step of generating, by using the physical property information, a self-organizing map on which each node is assigned a position in a map space and a physical property vector indicating a value related to a physical property quantity for each of a plurality of types of physical properties of the product;
a specification assignment step of assigning a specification vector indicating a value related to a material specification to each of the nodes by using the material specification information;
a target node detection step of acquiring target information indicating a desired physical property of the product and detecting, as a target node, the node to which the physical property vector matching a desired physical property indicated by the target information is assigned; and
recommendation data generation step of generating, by using the specification vector assigned to the target node, recommendation data representing the material specification of the material with which a product having the desired physical property can be generated.

8. The control method according to claim 7,
wherein in the recommendation data generation step, selecting at least one of the nodes from a sub-area in which the target node is included in the map space, and further generating the recommendation data for each of the selected nodes by using the specification vector assigned to that node.

9. The control method according to claim 8,
wherein in the recommendation data generation step, using, as the sub-area, an area whose center is at the target node and in which a distance from the target node is equal to or shorter than a threshold.

10. The control method according to claim 8,
wherein in the recommendation data generation means, performing:
dividing the map space into a plurality of clusters based on the specification vectors assigned to the respective nodes; and
using the cluster to which the target node belongs as the sub-area.

11. The control method according to claim 7,
wherein in the recommendation data generation step, further generating the recommendation data for each of a predetermined number of nodes in an ascending order of the distance from the target node in the map space by using the specification vector assigned to the node.

12. The control method according to any one of claims 7 to 11, further comprising:
a simulator control step of causing, for the product that can be generated by using a material specified by the material specification being input, a simulator to perform a simulation taking the material specification represented by the recommendation data as an input, the simulator being configured to generate prediction data of a physical property of the product; and
a simulation result output step of acquiring the prediction data generated by the simulator and outputting the prediction data and the recommendation data.

13. A non-transitory computer readable medium for causing a computer to perform:
an acquisition step of acquiring, for each of a plurality of patterns of a material that can be used in a target process, material specification information representing a material specification of the material and physical property information indicating a physical property quantity for each of a plurality of physical properties of a product that can be generated in the target process by using the material;
a self-organizing map generation step of generating, by using the physical property information, a self-organizing map on which each node is assigned a position in a map space and a physical property vector indicating a value related to a physical property quantity for each of a plurality of types of physical properties of the product;
a specification assignment step of assigning a specification vector indicating a value related to a material specification to each of the nodes by using the material specification information;
a target node detection step of acquiring target information indicating a desired physical property of the product and detecting, as a target node, the node to which the physical property vector matching a desired physical property indicated by the target information is assigned; and
recommendation data generation step of generating, by using the specification vector assigned to the target node, recommendation data representing the material specification of the material with which a product having the desired physical property can be generated.

14. The non-transitory computer readable medium according to claim 13,
wherein in the recommendation data generation step, selecting at least one of the nodes from a sub-area in which the target node is included in the map space, and further generating the recommendation data for each of the selected nodes by using the specification vector assigned to that node.

15. The non-transitory computer readable medium according to claim 14,
wherein in the recommendation data generation step, using, as the sub-area, an area whose center is at the target node and in which a distance from the target node is equal to or shorter than a threshold.

16. The non-transitory computer readable medium according to claim 14,
wherein in the recommendation data generation means, performing:
dividing the map space into a plurality of clusters based on the specification vectors assigned to the respective nodes; and
using the cluster to which the target node belongs as the sub-area.

17. The non-transitory computer readable medium according to claim 13,
wherein in the recommendation data generation step, further generating the recommendation data for each of a predetermined number of nodes in an ascending order of the distance from the target node in the map space by using the specification vector assigned to the node.

18. The non-transitory computer readable medium according to any one of claims 13 to 17, further comprising:
a simulator control step of causing, for the product that can be generated by using a material specified by the material specification being input, a simulator to perform a simulation taking the material specification represented by the recommendation data as an input, the simulator being configured to generate prediction data of a physical property of the product; and
a simulation result output step of acquiring the prediction data generated by the simulator and outputting the prediction data and the recommendation data.
